# EUROPÄISCHE PATENTSCHRIFT

(11) **EP 3 473 240 B1**
(45) Veröffentlichungstag und Bekanntmachung des Hinweises auf die Patenterteilung: **25.12.2019**
(21) Anmeldenummer: 17196964.5
(22) Anmeldetag: 18.10.2017
(51) Int. Cl.: A61K 9/00, A61K 9/10, A61K 9/48, A23L 33/115

(54) **EINE IM WESENTLICHEN WASSERFREIE ZUSAMMENSETZUNG, INSBESONDERE ZUR VERWENDUNG ALS NAHRUNGSERGÄNZUNGSMITTEL**
A PRIMARILY ANHYDROUS COMPOSITION, IN PARTICULAR FOR USE AS NUTRITIONAL SUPPLEMENTS
COMPOSITION ESSENTIELLEMENT SANS EAU, EN PARTICULIER POUR L'UTILISATION EN TANT QUE COMPLÉMENT ALIMENTAIRE

(43) Veröffentlichungstag der Anmeldung: 24.04.2019
(73) Patentinhaber: Qineva GmbH, 92521 Schwarzenfeld (DE)
(72) Erfinder: Richter, Werner Oskar, 91257 Pegnitz (DE)
(74) Vertreter: Maikowski & Ninnemann Patentanwälte Partnerschaft mbB

(56) Entgegenhaltungen:
- WO-A1-2016/135482
- AU-A4- 2009 100 934
- GIGLIOTTI J C ET AL: "Extraction and characterisation of lipids from Antarctic krill (Euphausia superba)", FOOD CHEMISTRY, ELSEVIER LTD, NL, Bd. 125, Nr. 3, 1. April 2011 (2011-04-01) , Seiten 1028-1036, XP027477867, ISSN: 0308-8146, DOI: 10.1016/J.FOODCHEM.2010.10.013 [gefunden am 2010-11-04]
- STINE M. ULVEN ET AL: "Metabolic Effects of Krill Oil are Essentially Similar to Those of Fish Oil but at Lower Dose of EPA and DHA, in Healthy Volunteers", LIPIDS, Bd. 46, Nr. 1, 1. Januar 2011 (2011-01-01) , Seiten 37-46, XP055108490, ISSN: 0024-4201, DOI: 10.1007/s11745-010-3490-4

## Beschreibung

Die vorliegende Erfindung betrifft eine im Wesentlichen wasserfreie Zusammensetzung, insbesondere zur Verwendung als Nahrungsergänzungsmittel und ein Verfahren zu deren Herstellung.

### Beschreibung

Die Aufnahme von fettlöslichen und bestimmten wasserlöslichen Nahrungsbestandteilen vom menschlichen Darm ist begrenzt. So werden fettlösliche Nahrungsbestandteile vom menschlichen Darm nur zu einem kleinen Prozentsatz aufgenommen. Dazu kommt, dass die Fettverdauung sehr anfällig ist gegenüber Störungen. Sie benötigt im Gegensatz zu anderen Nahrungsbestandteilen die gesamte Länge des Darms und erfordert Enzyme aus dem Mund, dem Magen und der Bauchspeicheldrüse sowie einen ausreichenden Gallefluss. Und die Passagezeit des Nahrungsbreis durch den Darm muss entsprechend angepasst sein, so dass die Enzyme auch genug Zeit haben, zu wirken. Daraus lassen sich vielfältige Gründe für eine nicht optimale Fettverdauung und damit für eine verminderte Aufnahme von fettlöslichen Nahrungsbestandteilen ableiten.

Auch die Aufnahme von bestimmten wasserlöslichen Komponenten, wie zum Beispiel Eisen, aus dem menschlichen Darm ist extrem störanfällig. Eisen wird zum allergrößten Teil nur in den ersten 30 cm des Dünndarms absorbiert.

Zur Verbesserung der Aufnahme von fettlöslichen oder wasserlöslichen Wirkstoffen im menschlichen Darm wurde vorgeschlagen, micellare Nanopartikel zu verwenden. So wird in der DE 10 2010 021688 A1 die Herstellung eines Wirkstoffkonzentrats aus micellaren Nanopartikeln beschrieben, wobei zunächst eine flüssige Voremulsion umfassend Glycerol und Phospholipide hergestellt und diese Voremulsion anschließend mit Wasser und einem Öl-Wirkstoff-Gemisch verrührt wird. Durch das Verrühren unter Anwendung von hohen Scherkräften werden Partikel in einer Größe von 750 nm bis 1250 nm erhalten. Durch eine anschließende Hochdruckhomogenisation entstehen Micellen im Nanobereich. Das gewonnene micellare Wirkstoffkonzentrat kann dann einer Ultrazentrifugation ausgesetzt werden, wobei eine erste Phase aus Glycerol und Wasser und eine zweite Phase aus einer öligen Micellenpaste,erhalten wird.

Die fertigen in der DE 10 2010 021 688 A1 beschriebenen Produkte weisen eine Wasseraktivität (a_{w}-Wert) von 0,78 bis 0,84 auf. Damit kann eine Abfüllung z. B. in Weich- oder Hartgelatinekapseln wegen der resultierenden Instabilität des Kapselmaterials nicht erfolgen. Die Einnahme muss in einer wässrigen Emulsion als Paste erfolgen. Damit ist die dauerhafte Anwendung aufgrund geschmacklicher Problematik kaum möglich.

Der vorliegenden Erfindung liegt daher die technische Aufgabe zugrunde, die aus dem Stand der Technik bekannten Nachteile zu verbessern und ein Produkt zur Verfügung zu stellen, welches eine effektive und dauerhaft problemlose Aufnahme von fettlöslichen und bestimmten wasserlöslichen Wirkstoffen in hohen Konzentrationen ermöglicht.

Die gestellte Aufgabe wird erfindungsgemäß durch eine Zusammensetzung mit den Merkmalen des Anspruchs 1 gelöst.

Entsprechend wird eine im Wesentlichen wasserfreie Zusammensetzung bereitgestellt, die folgende Komponenten umfasst:
- mittels Ultrazentrifugation vorbehandeltes Krillöl, wobei das Krillöl mittels Ultrazentrifugation bei 200.000 bis 250.000 g, bevorzugt 210.000 bis 240.000 g für 20 bis 30 Minuten vorbehandelt wird,
- optional Silica als Emulsionsmittel,
- mindestens einen wasserlöslichen Wirkstoff und/oder mindestens einen fettlöslichen Wirkstoff, und
- mindestens eine antioxidative Substanz.

"Im Wesentlichen wasserfrei" bedeutet im Sinne der vorliegenden Zusammensetzung, dass der Wassergehalt so gering ist, dass ein Wachstum von Mikroorganismen, insbesondere von Pilzen und Bakterien nicht unterstützt wird und ausgeschlossen ist. So weist die vorliegende Zusammensetzung in einer Ausführungsform eine Wasseraktivität a_{w} zwischen 0,2 und 0,5, bevorzugt zwischen 0,25 und 0,4 auf. Eine Abfüllung in Weich- oder Hartgelatinekapseln ist damit möglich.

Das bei der Herstellung der vorliegenden Zusammensetzung verwendete Krillöl ist ein aus dem antarktischen Krill (*Euphausia superba*) extrahiertes Öl. Das verwendete Krillöl besteht durchschnittlich zu 44,7 % aus neutralen Lipiden und zu 44 % aus polaren Lipiden. Bei den polaren Lipiden dominiert Phosphatidylcholin (> 86 %). Damit ist es eines der wenigen Öle mit einem hohen Anteil an Phospholipiden. Zum Vergleich: Sojaöl enthält 2 % Phospholipide.

Entsprechend umfasst die vorliegende Zusammensetzung Phospholipide, insbesondere Phosphatidylcholin, die im Krillöl enthalten sind, und ggf. aus diesem gewonnen werden.

Wie oben erwähnt, wird das Krillöl vor Verwendung mittels Ultrazentrifugation vorbehandelt. Erfindungsgemäß umfasst die Vorbehandlung eine Ultrazentrifugation bei 200.000 bis 250.000 g, bevorzugt 210.000 bis 240.000 g für 20 bis 30 Minuten bei 30°C.

Das derart vorbehandelte Krillöl (d.h. einem hohen Scherstress ausgesetzte Krillöl) ist im Gegensatz zum nativen Krillöl sehr schnell und leicht (in wenigen Sekunden) vollständig in Wasser emulgierbar. Dies ist Voraussetzung für eine optimale Verfügbarkeit im Magen.

In einer alternativen Ausführungsform wird dem nativen Krillöl vor der Vorbehandlung mittels Ultrazentrifugation Wasser (z.B. pro ml Krillöl zwischen 20 und 100 µl steriles Wasser) zugegeben. In Folge der Ultrazentrifugation setzen sich die im Krillöl enthaltenden Phospholipide als fester Unterstand (Feststoff) ab und können durch Abgießen des öligen Überstands gewonnen werden. Der Wassergehalt des Unterstands kann durch die g Zahl bei der Ultrazentrifugation und die anschließende Behandlung mit Wärme bedarfsgemäß reguliert werden. Die komplette Emulgierung des Unterstandes in verschiedene dünnflüssige Öle (z. B. Kokosöl) oder Wasser ist gegeben.

In wässrigem Milieu bildet das Krillöl, genauer die im Krillöl enthaltenen Phospholipide, Liposomen mit einem Durchmesser zwischen 50 und 100 Nanometern. Weitere Stoffe oder Komponenten zur Stabilisierung der gebildeten Liposomen sind nicht vorgesehen.

Im Falle eines fettlöslichen Wirkstoffes wird dieser in die gebildete Membran des Partikels aufgenommen. Vorteil ist, dass so kleine Partikel eine hohe Affinität zu Oberflächen haben, sich also an die Schleimhaut von Magen und Darm anlagern. Nur durch den Kontakt mit der Schleimhaut können fettlösliche Stoffe in den Blutkreislauf kommen. Die Bildung der Liposomen wird den natürlichen Vorgängen im Magen und Darm überlassen. Die normale menschliche Fettverdauung erfordert "Liposomen", die einen Durchmesser von im Mittel 65 nm haben. Zur Bildung benötigt man die volle Funktion von Enzymen aus Mund, Magen und Bauchspeicheldrüse, einen ausreichenden Gallefluss und eine funktionierende Magenmuskulatur (um die Nahrung zu zerkleinern). Die Produkte übernehmen daher für die Aufnahme der fettlöslichen Stoffe diese Funktionen.

Darüber hinaus folgen die durch das Krillöl eingebrachten Triglyceride dem normalen Fettabbauweg und bringen darüber fettlösliche Stoffe, z. B. Coenzym Q10, in das Blut.

Das Folgende tritt nur ein, wenn dem Produkt mittelkettige Triglyceride zugesetzt werden, z. B. aus Kokosöl. Die isomerisierten Diglyceride, die durch Einwirkung der Magenlipase und der Magensäure im Magen entstehen, werden darüber hinaus mit einer zeitlichen Verzögerung aus dem Darm aufgenommen, so dass dann ggf. über Stunden noch fettlösliche Stoffe aus dem Darm aufgenommen werden können.

Im Falle von bestimmten wasserlöslichen Wirkstoffen, wie z. B. Eisen in Form von Eisen-II-Sulfat, binden sich diese direkt an die Phospholipide. Diese bilden dann durch das wässrige Milieu im Magen Liposomen mit hoher Affinität zur Dünndarmschleimhaut. Das Eisen lagert sich also mit den Liposomen an die Schleimhaut im ersten Bereich des Dünndarms an, um dort mit hoher Effektivität in das Blut aufgenommen zu werden. Daher ist eine deutlich niedrigere Dosierung als in herkömmlichen Produkten möglich, so dass die gefürchteten subjektiven Nebenwirkungen im Magen- und Darmbereich nicht auftreten.

In einer bevorzugten Ausführungsform weist die vorliegende Zusammensetzung Phospholipide, insbesondere das Phosphatidylcholin, in einer Menge zwischen 20 und 35 Gew%, bevorzugt zwischen 23 und 33,5 Gew% auf.

In einer weiteren Ausführungsform enthält die vorliegende Zusammensetzung Silica (Siliziumdioxid) in einer Menge zwischen 5 bis 15 Gew%, bevorzugt zwischen 6 bis 10 Gew% bezogen auf die Gesamtmenge. Die verwendete Menge an Silica kann entsprechend 30 - 36 mg (5,1-6,2 Gew%) betragen. Bevorzugt wird Silica mit einem mittleren Durchmesser von 70 bis 90 µm, insbesondere 75 µm, und einem minimalen Durchmesser von 5 bis 15µm, insbesondere 10 µm verwendet.

Wie oben bereits angeführt, dient Silica bzw. Silicagel als Emulsionsmittel und ermöglicht eine gute Vermischung und Verteilung der Komponenten der Zusammensetzung; die Durchmischung der Bestandteile der Zusammensetzung wird verbessert. Dies trifft insbesondere auf wässrige Zubereitungen (z. B. Wasser oder Orangensaft) zu. Dies ist die Situation bei Einnahme des Produktes mit Flüssigkeit im Magen. Bei der in-vitro Suspension in Wasser bilden sich Nanopartikel mit einer durchschnittlichen Größe von typischerweise 65 - 85 nm. Die entstehenden Nanopartikel bestehen aus Phospholipiden und bilden eine physiologische Form der Verabreichung.

Wie oben erwähnt, umfasst die vorliegende Zusammensetzung fettlösliche oder wasserlösliche Wirkstoffe. Als Wirkstoffe werden dabei Substanzen bezeichnet, die in menschlichen Organismus eine spezifische Wirkung haben und eine spezifische Reaktion hervorrufen. Wirkstoffe werden in folgenden Bedeutungen verwendet: Nahrungsbestandteile oder arzneilich wirksame Substanzen.

Der in der vorliegenden Zusammensetzung verwendete wasserlösliche Wirkstoff umfasst bevorzugt Metallkationen und/oder organische Kationen. Als Metallkationen werden bevorzugt Eisenkationen oder auch Selenkationen verwendet. Im Falle von Eisen wird dieses in Form von Eisen(II)Sulfat zugegeben.

Als organische Kationen können zum Beispiel Kationen des Chondroitinsulfats verwendet werden.

Der wasserlösliche Wirkstoff ist in der vorliegenden finalen Zusammensetzung in einer Menge zwischen 0,05 und 10 Gew%, bevorzugt zwischen.0,2 und 8,6 Gew% enthalten.

Der in der vorliegenden Zusammensetzung verwendete fettlösliche Wirkstoff umfasst bevorzugt fettlösliche Nahrungsbestandteile oder Arzneistoffe. So kann der fettlösliche Wirkstoff ausgewählt sein aus der Gruppe enthaltend Coenzym Q10, alpha-Carotin, Curcumin, Naringin, Olivenölflavonoide oder Lycopin.

Der fettlösliche Wirkstoff ist in der vorliegenden finalen Zusammensetzung in einer Menge zwischen 5,0 und 25 Gew%, bevorzugt 10 bis 23,0 Gew%, z.B. 8,6 - 23 Gew% enthalten. Somit ermöglicht die vorliegende Zusammensetzung die Verabreichung einer relativ hohen Menge an Wirkstoffen.

Wie oben angeführt, kann die vorliegende Zusammensetzung mindestens eine fettlösliche antioxidative Substanz enthalten. Diese fettlösliche antioxidative Substanz kann ausgewählt sein aus einer Gruppe enthaltend Substanzen mit Vitamin-E-Aktivität, insbesondere Tocopherole und Trienole, sowie Carotinoide.

Tocopherole oder Derivate davon sind insbesondere alpha-Tocopherol, beta- und gamma-Tocopherol, delta-Tocopherol. Trienole sind insbesondere alpha-Trienol, beta-Trienol, gamma-Trienol und delta-Trienol.

Carotinoide sind eine bevorzugte Gruppe von Antioxidantien, die in der vorliegenden Erfindung verwendet werden können. Diese Antioxidantien sind eine Klasse von Verbindungen, die in zwei Hauptgruppen eingeteilt werden: Carotine und Xanthophylle. Im Gegensatz zu Carotinen, die reine Polyen-Kohlenwasserstoffe sind wie Beta-Carotin oder Lycopin, enthalten Xanthophylle funktionelle Sauerstoffgruppen, wie Hydroxyl-, Epoxy- und/oder Oxogruppen. Typische Beispiele für die Xanthophyllgruppe sind Astaxanthin, Canthaxanthin, Lutein, Zeaxanthin und Fucoxanthin, Natürliche Quellen von Astaxanthin sind Krill, Langusten, Nebenprodukte der Krustentierverarbeitung, Bakterien, Hefen, Algen und höheren Pflanzen.

Mögliche Carotinoide sind Alpha-Carotin, Beta-Carotin, Lutein, Zeaxanthin, Retinal, Astaxanthin, Cryptoxanthin, natürliche gemischte Carotinoide, Lycopin und Resveratrol.

Besonders bevorzugt werden Lutein und Zeaxanthin verwendet, die neben den antioxidativen Eigenschaften auch einer Degeneration der Netzhaut des Auges und damit der Sehkraft entgegenwirken sollen.

Die Konzentrationen der Carotinoide in den Formulierungen können variieren, entsprechen aber solchen Mengen, die in Nahrungsergänzungsmitteln oder ergänzend bilanzierten Diäten nützlich sind.

Neben den bereits angeführten Komponenten kann die vorliegende Zusammensetzung auch weitere Inhaltsstoffe aufweisen. Weitere Bestandteile, die in der Formulierung gemäß der vorliegenden Erfindung vorliegen können, sind Vitamine, wie Vitamin A, Vitamin-A-Acetat, Vitamin-A-Palmitat, Riboflavin, Vitamin B, Ascorbinsäure, Ascorbylpalmitat, Nicotinsäure, Nicotinamid, Pyridoxin-Hydrochlorid, Vitamin D3, Vitamin K, Thiamin, Calcium-Pantothenat, Biotin, alpha-Liponsäure, Verbindungen mit Vitamin- oder Coenzym Eigenschaften wie Cholinchlorid, Carnitin, Taurin, Kreatin, Ubichinone, S-Methyl- und S-Adenosylmethionin. Diese Vitamine und Substanzen können gegebenenfalls entweder als Antioxidantien gemäß der vorliegenden Erfindung oder als weitere Faktoren zur Unterstützung eines oder mehrerer Bestandteile der Formulierung gemäß der vorliegenden Erfindung verwendet werden oder einfach als weitere vorteilhafte Inhaltsstoffe funktionieren.

Bevorzugt wird Vitamin D3 zugesetzt. Wenn Vitamin D3 vorhanden ist, ist es vorzugsweise in einer Konzentration von 0,000017 - 0,000034 Gew% in der finalen Zusammensetzung enthalten.

Gegebenenfalls kann die Formulierung gemäß der Erfindung auch physiologische akzeptable Hilfsstoffe umfassen wie beispielsweise Lecithin, Mono-, Glycerinmonostearat, Kokos- und Sojaöl.

Kokosöl enthält ca. 50 % sogenannte mittelkettige Fettsäuren. Diese haben die Eigenschaft, dass sie für die Aufnahme in die Blutbahn nur ein Enzym aus dem Magen benötigen, also leichter verdaulich sind. Ihre Aufnahme kann im Magen und im gesamten Darm erfolgen. Sie finden sich im Triglycerid (dem klassischen Fett) immer randständig (Position 1 oder 3). Ihre Abspaltung kann einerseits zu einer Resorption von fettlöslichen Stoffen führen, andererseits kommt es dann durch den Einfluss der Magensäure zu einer Isomerisierung (von 2-3- oder 1-2-Diglyceriden zu 1-3-Diglyceriden). Dies beeinflusst die weitere Fettaufnahme aus dem Darm.

Sojaöl dient als Lösungsmittel für die fettlöslichen antioxidativen Substanzen, wie Tocopherole und Trienole.

In einer bevorzugten Variante umfasst die vorliegende Zusammensetzung aus Krillöl gewonnene Phospholipide, insbesondere Phosphatidylcholin, Silica, Eisen(II)sulfat, Tocopherole und Sojaöl. In einer besonders bevorzugten Variante umfasst die Zusammensetzung aus Krillöl gewonnene Phospholipide, insbesondere Phosphatidylcholin, Silica, Eisen(II)sulfat, Sojaöl, alpha-Tocopherol, beta- und gamma-Tocopherol sowie delta-Tocopherol.

In einer weiteren bevorzugten Variante umfasst die vorliegende Zusammensetzung aus Krillöl gewonnene Phospholipide, insbesondere Phosphatidylcholin, Silica, Coenzym Q10, Tocopherole, Trienole, Sojaöl und Carotinoide. In einer besonders bevorzugten Variante umfasst die Zusammensetzung aus Krillöl gewonnene Phospholipide, insbesondere Phosphatidylcholin, Silica, Coenzym Q10, Sojaöl, alpha-Tocopherol, beta- und gamma-Tocopherol, delta-Tocopherol, alpha-Trienol, beta-Trienol, gamma-Trienol und delta-Trienol, Lutein, Zeaxanthin und Vitamin D3.

Nach Zusammenmischen der Inhaltsstoffe kann die Zusammensetzung einer Ultrazentrifugation (bei 225 000 g) unterzogen werden, wobei eine ölige Paste entsteht, die mit Wasser oder einer anderen Flüssigkeit eingenommen werden kann. Alternativ kann es auch in andere Produkte eingebracht werden.

Noch bevorzugter wird die Zusammensetzung jedoch einer Differentialtemperaturzentrifugation ausgesetzt und die so erhaltene fluide Suspension in Kapseln abgefüllt.

Entsprechend wird gemäß der vorliegenden Erfindung eine Kapsel bereitgestellt, die die erfindungsgemäße Zusammensetzung enthält. Die Kapselhülle kann aus Hart- oder Weichgelatine bestehen. Eine Kapsel kann zwischen 500 und 1000 mg, bevorzugt zwischen 550 und 750 mg, insbesondere 580 mg der erfindungsgemäßen Zusammensetzung enthalten.

Die Aufgabe der vorliegenden Erfindung wird ebenfalls mit einem Verfahren zur Herstellung der erfindungsgemäßen Zusammensetzung gelöst, welches die folgenden Schritte umfasst:
- Vorbehandeln von Krillöl mittels Ultrazentrifugation bei 200.000 bis 250.000 g, bevorzugt 210.000 bis 240.000 g für 20 bis 30 Minuten;
- Vermischen des vorbehandelten Krillöls mit mindestens einem wasserlöslichen Wirkstoff und/oder mindestens einem fettlöslichen Wirkstoff;
- Zugabe von Silica und mindestens einer antioxidativen Substanz,
- Rühren der Mischung aus vorbehandeltem Krillöl, wasserlöslichem Wirkstoff und/oder
   fettlöslichem Wirkstoff, Silica und antioxidativer Substanz über einen vorgegebenen Zeitraum, und
- Zentrifugation der Mischung bei 14.000 bis 16.000 g bei gleichzeitiger Absenkung der Temperatur der Mischung auf einen Wert zwischen 3°C und 10°C, bevorzugt zwischen 5°C und 8°C.

Wie bereits oben beschrieben, umfasst die Vorbehandlung des Krillöls eine Ultrazentrifugation bei 200.000 bis 250.000 g, bevorzugt 210.000 bis 240.000 g für 20 bis 30 Minuten bei 30 °C. Entweder wird das Krillöl als solches einer Ultrazentrifugation ausgesetzt oder es wird dem Krillöl vor Ultrazentrifugation etwas Wasser zugegeben, um eine Abscheidung der im Krillöl enthaltenden Phospholipide zu bewirken.

Durch die spezifischen Zentrifugationsbedingungen wird eine fluide Suspension (aus einer vor der Zentrifugation im Wesentlichen festen Zusammensetzung) erhalten, die in Kapseln abgefüllt werden kann. Entsprechend kann durch die spezifische Zentrifugationstechnik die Viskosität der Zubereitung vermindert werden.

Gemäß einer Variante des Verfahrens wird das Krillöl vor dem Vermischen mit mindestens einem Wirkstoff, bevorzugt in einer Inertgas-Atmosphäre, auf eine Temperatur zwischen 40 °C und 90°C bevorzugt zwischen 50 °C und 80 °C erwärmt.

Die Absenkung der Temperatur während der Zentrifugation (Differentialtemperaturzentrifugation) erfolgt bevorzugt von einer Ausgangstemperatur von 80 °C auf 5 °C.

Folgende Ausgangsformulierungen (vor der Zentrifugation) werden bevorzugt verwendet (jeweils bezogen auf das vorbehandelte Krillöl):
- 5 bis 15 Gew%, bevorzugt 6 bis 10 Gew% Silica;
- 5 bis 25 Gew%, bevorzugt 10 bis 23 Gew% an fettlöslichen Wirkstoffen und/oder 0,05 bis 20 Gew%, bevorzugt 0,1 bis 10 Gew%, insbesondere bevorzugt 0,2 bis 8,6 Gew% an wasserlöslichen Wirkstoffen; und
- Rest an antioxidativen Substanzen und weiteren Bestandteilen.

In einer Ausführungsform umfasst die Ausgangsformulierung z.B. 18 Gew% Eisen(II)sulfat-heptahydrat, 6 Gew% Silica, 4 Gew% Sojaöl und 4 Gew% Tocopherole bezogen auf die Gesamtmenge des Krillöls.

In einer weiteren Ausführungsform umfasst die Ausgangsformulierung z.B. 20 Gew% Coenzym Q10, 10 Gew% Silica, 6 Gew% Sojaöl, 3 Gew% Tocopherole, 1,5 Gew% Trienole bezogen auf die Gesamtmenge des Krillöls.

Die Erfindung wird nachfolgend anhand von Ausführungsbeispielen näher erläutert.

### Ausführungsbeispiel 1: Vorbehandlung des Krillöls

a) Ultrazentrifugation des Krillöls bei 235 000 g bei 30°C über 20 - 30 Minuten (Beckman Coulter Optima L-90K Ultrazentrifuge - Rotor 45 Ti).
b) Zum nativen Krillöl werden pro ml zwischen 20 und 100 µl steriles Wasser gegeben, intensiv gemischt und dann einer Ultrazentrifugation mit 210 000 g bei 30 °C über 25 - 30 Minuten unterzogen (Beckman Coulter Optima L-90K Ultrazentrifuge - Rotor 45 Ti).

### Ausführungsbeispiel 2: Erste Zusammensetzung mit Coenzym Q10

Diese Zusammensetzung zielt darauf ab, die im Alter besonders wichtigen Funktionen Hirnleistungsfähigkeit, Muskel- und Sehkraft zu unterstützen. Bei den Inhaltsstoffen handelt es sich um fettlösliche Substanzen, die üblicherweise nur zu einem kleinen Prozentsatz aus dem Darm des Menschen aufgenommen werden.

Vorbehandeltes Krillöl wird unter Lichtabschirmung und Begasung mit 99,9 % Stickstoff bei 80 °C mittels eines dreiflügeligen Düsenrührers mit 6,3 Ncm 20 Minuten lang durchmischt. Dann werden unter stetigem Rühren pro g Krillöl 119 mg Coenzym Q10 als Pulver zugesetzt. Nach vollständigem Einrühren des Coenzym Q10 werden 47 mg Silica, 29 mg Sojaöl, 1,6 mg alpha-Tocopherol, 13,5 mg beta- und gamma-Tocopherol sowie 5,8 mg delta-Tocopherol zugesetzt. Nach vollständiger Mischung folgen 47 mg Silica, 29 mg Sojaöl, 5,8 mg alpha-Tocopherol, 5 mg alpha-Trienol, 0,6 mg beta-Trienol, 7,2 mg gamma-Trienol und 2,0 mg delta-Trienol. Dann erfolgen in dieser Reihenfolge die Zugaben von 12 mg Lutein, 24 mg Zeaxanthin und 46 µg Vitamin D3.

Nach 20-minütigem Rühren unter den oben genannten Bedingungen erfolgt eine Differentialtemperaturzentrifugation, bei der während 30 Minuten Zentrifugierens bei 15 970 g die Temperatur kontinuierlich von 80 °C auf 5 °C abgesenkt wird. Die dann erhaltende fluide Suspension kann in Hartgelatinekapseln abgefüllt werden. Der a_{w}-Wert liegt bei 0,283. Keimwachstum ist daher nicht möglich.

Alternativ kann durch Ultrazentrifugation bei 235 000 g eine Paste mit den Inhaltsstoffen gewonnen und beliebig weiter verarbeitet werden.

Bei Suspension in Wasser (dem Magen entsprechend) finden sich Partikel mit einer Größe von im Mittel 59 nm.

Resorptionsuntersuchungen wurden bei 8 Personen einvernehmlich durchgeführt. Blut wurde vor Einnahme der Zusammensetzung und nach 15 Minuten, 1 Stunde, 2 Stunden, 4 Stunden und 6 Stunden durchgeführt. Durch Analyse von Coenzym Q10 mit Hochdruckflüssigkeitschromatographie oder ELISA konnte die Resorption bereits nach 15 Minuten und über den gesamten Zeitraum nachgewiesen werden. Mit einem herkömmlichen Produkt in Form eines Coenzym-Q10-Pulver war dies nicht möglich.

Zur Analyse erfolgte die Auftrennung des Blutserums mittels Ultrazentrifugation in Very-Iowdensity-Lipoproteine, High-density- und Low-density-Lipoproteine und Lipoprotein-freies Serum. Dabei fand sich der Anstieg der Coenyzm-Q-10-Konzentration in der Fraktion mit einer Dichte von < 1,006 g/ml. Phospholipide hingegen haben bei 18°C eine Dichte von > 1,006 g/ml. Daraus kann geschlossen werden, dass sich das vorliegende Produkt der üblichen regulierten Aufnahmewege für Coenzym Q10 bedient und eine unspezifische Aufnahme über Nanopartikel, die durch die Darmwand penetrieren nicht stattfindet. Die Phospholipide werden erwartungsgemäß vor der Absorption im Darm gespalten und damit die Nanopartikelstruktur zerstört.

Coenzym Q10 besitzt im Körper des Menschen kein eigenes Transportsystem, sondern bedient sich des Fettstoffwechsels. Daher besteht eine enge Korrelation zwischen der Höhe der Blutfettparameter und den Coenzym Q10-Blutkonzentrationen. Dies bedeutet aber auch, dass die Höhe des Coenzym Q10-Spiegels sich bei Fettstoffwechselstörungen ändert, ohne dass dies etwas mit der Resorption zu tun hat. So weisen Personen, die nach dem Essen viele Triglyceride aus der Leber freisetzen, sehr hohe Konzentrationen auf. Andererseits haben jene, welche die aus dem Essen anflutenden Fettsäuren in der Leber komplett verbrennen, sehr niedrige Konzentrationen. Ohne exakte Kenntnis des Fettstoffwechsels, für welche die Bestimmung von Cholesterin, Triglyceriden, HDL- und LDL-Cholesterin nicht ausreicht, kann daher die Bioverfügbarkeit von Coenzym Q10 nicht beurteilt werden.

### Ausführungsbeispiel 3: Zweite Zusammensetzung mit Coenzym Q10

Vorbehandeltes Krillöl wird unter Lichtabschirmung und Begasung mit 99,9 % Stickstoff bei 80 °C mittels eines dreiflügeligen Düsenrührers mit 6,3 Ncm 20 Minuten lang durchmischt. Dann werden unter stetigem Rühren pro g Krillöl 119 mg Coenzym Q10 als Pulver zugesetzt. Nach vollständigem Einrühren des Coenzym Q10 werden 47 mg Silica, 29 mg Sojaöl, 1,6 mg alpha-Tocopherol, 13,5 mg beta- und gamma-Tocopherol sowie 5,8 mg delta-Tocopherol zugesetzt. Nach 20 minütigem Rühren unter den oben genannten Bedingungen erfolgt eine Differentialtemperaturzentrifugation, bei der während 30 Minuten Zentrifugieren bei 15 970 g die Temperatur kontinuierlich von 80 °C auf 5 °C abgesenkt wird.

Die dann erhaltende fluide Suspension kann in Hartgelatinekapseln abgefüllt werden. Der a_{w}-Wert liegt bei 0,460. Bakterien- oder Pilzwachstum ist daher nicht möglich.

Alternativ kann durch Ultrazentrifugation bei 235 000 g eine Paste mit den Inhaltsstoffen gewonnen und beliebig weiter verarbeitet werden, z. B. durch die Zugabe von Kokosöl.

Bei Verdünnung in Wasser ergibt sich eine Partikelverteilung mit einer mittleren Größe von ca. 60 nm.

### Ausführungsbeispiel 4: Zusammensetzung mit Eisen

Krillöl wird in Dunkelheit unter Stickstoffatmosphäre bei einer Temperatur von 48 °C 20 Minuten mit 6,3 Ncm gerührt. Dann werden 104 mg Eisen-II-Sulfat-Heptahydrat (bearbeitet mit einem Homogenisator) pro 393 mg Krillöl langsam eingerührt.

Fünf Minuten später werden hinzugefügt, ebenfalls zu ursprünglich 393 mg Krillöl:
33 mg Silica (mittlerer Durchmesser 75 µm, minimaler Durchmesser 10 µm)
24 mg Sojaöl
2 mg alpha-Tocopherol
17 mg beta- und gamma-Tocopherol
7 mg delta-Tocopherol

Eisen-II-Sulfat-Heptahydrat bindet an Phospholipide und kann durch Zentrifugation bei 14 500 g angereichert werden. Durch Ultrazentrifugation bei 235 000 g kann eine wasserfreie Masse aus Phospholipiden und Eisen-II-Sulfat-Heptahydrat hergestellt werden. Diese ist zur Weiterverarbeitung zu verschiedenen Produkten, z. B. in Nahrungsmitteln, geeignet.

Silica wird zugegeben, um die Emulgierbarkeit des Krillöls in einer wässrigen Lösung (Einnahme des Produkts mit einem Glas Wasser oder Orangensaft (Vitamin C)) zu erleichtern. Bei der in-vitro Suspension in Wasser bilden sich dabei Nanopartikel mit einer durchschnittlichen Größe von 65 nm. Die Tocopherole werden in Sojaöl gelöst und dienen als zusätzlicher Oxidationsschutz für die verschiedenen enthaltenen Fettsäuren.

Der a_{w}-Wert des Produktes liegt bei 0,420. Bakterien- oder Pilzwachstum ist bei dieser Wasseraktivität auszuschließen. Die Abfüllung in Weich- oder Hartgelatinekapseln ist möglich.

Durch die Bindung an Phosphatidylcholin und die physiologische Entstehung von Nanopartikeln in wässriger Lösung kann die Aufnahme von Eisen gesteigert werden. Dabei wurden im Rahmen von Heilversuchen durch einen Arzt 10 Personen evaluiert, die mit anderen Eisenprodukten nicht wirksam behandelt werden konnten.

Bei den 10 Probanden im Rahmen des Heilversuchs wurde morgens um 9.00 h nüchtern eine Hartgelatinekapsel mit 20 mg zweiwertigem Eisen verabreicht. Blut wurde aus einer Armvene unmittelbar vor der Einnahme der Kapsel sowie nach 1 Stunde, 2 Stunden und 4 Stunden entnommen.

Die folgende Tabelle zeigt in µg/dl die basale Konzentration und die maximale Konzentration im Rahmen der Eisenbelastung. Die Messung der Eisenkonzentrationen erfolgte mit einem AU 400 Beckman Coulter Analyzer.

| Person | Basale Konzentration [µg/dl] | Maximale Konzentration [µg/dl] |
|---|---|---|
| 1 | 67 | 192 |
| 2 | 40 | 185 |
| 3 | 64 | 183 |
| 4 | 83 | 194 |
| 5 | 34 | 137 |
| 6 | 27 | 209 |
| 7 | 50 | 143 |
| 8 | 77 | 185 |
| 9 | 79 | 207 |
| 10 | 56 | 154 |
| *Mittelwert* | *57,7* | *178,9* |
| *Standardabweichung* | *19,6* | *25,4* |

Die Behebung des Eisenmangels konnte durch Bestimmung der Speicherform des Eisens, Ferritin, belegt werden. Nebenwirkungen in Form von Übelkeit, Oberbauchschmerzen, Blähungen, Verstopfung, Durchfall oder schwarzem Stuhl wurden nicht beobachtet. Alle Patienten gaben eine Besserung der klinischen Beschwerden durch den Eisenmangel in wenigen Tagen an. Im Vergleich zur oben beschriebenen Zubereitung als Paste (DE 10 2010 021688 A1) war die Aufnahme von Eisen im Eisenresorptionstest deutlich höher. Der maximale Anstieg mit der Paste (10 mg) hatte im Mittel 43 µg/dl betragen (Patienten mit Eisenmangel).

Darüber hinaus wurden 12 Personen ohne Eisenmangel (Ferritin > 30 ng/ml) einem Eisenresorptionstest unterzogen, um festzustellen, ob die über Hepcidin regulierte Aufnahme von anorganischem Eisen aus dem Darm erhalten bleibt. Dadurch konnte festgestellt werden, dass die Aufnahme bei fehlendem Eisenmangel deutlich niedriger ist. Je höher das Speichereisen (Ferritin), desto geringer die Aufnahme. Eine Überladung des Körpers mit Eisen durch das Produkt ist daher nicht möglich.

Die folgende Tabelle zeigt die Ergebnisse von Eisenresorptionstests aus der Literatur im Vergleich mit dem eigenen Produkt:

| ***Eisenart*** | ***Eisenmenge*** | ***Anstieg der Blutserumkonzentration*** | ***n*** |
|---|---|---|---|
| Eisensulfat | 105 mg | 158 µg/dl | 20 |
| Eisenfumarat | 65 mg | 100 µg/dl (F)/ 67 µg/dl (M) | 49 |
| Eisensulfat | 100 mg | 93 µg/dl | 10 |
| Eisensulfat | 80 mg | 86 µg/dl °/164 µg/dl°° | 69 |
| Eisensulfat | 200 mg | 114 µg/dl | 111 |
| Eisensulfat | 10 mg | 28 µg/dl /F)/17 µg/dl (M) | 122 |
| **Eisensulfat** | **20 mg** | **121 µg/dl*/65 µg/dl**** | **22** |

| | | | |
|---|---|---|---|
| F = Frauen, M = Männer * Personen mit Eisenmangel (n = 10 - 8 Frauen, 2 Männer, 29 - 83 Jahre alt) ** Personen ohne Eisenmangel (n = 12) ° Personen älter als 65 Jahre mit Eisenmangelblutarmut °° Personen jünger als 65 Jahre mit Eisenmangelblutarmut | | | |

## Patentansprüche

1. Eine im Wesentlichen wasserfreie Zusammensetzung umfassend
- mittels Ultrazentrifugation vorbehandeltes Krillöl, wobei das Krillöl mittels Ultrazentrifugation bei 200.000 bis 250.000 g für 20 bis 30 Minuten vorbehandelt wird.
- optional Silica als Emulsionsmittel,
- mindestens einen wasserlöslichen Wirkstoff und/oder mindestens einen fettlöslichen Wirkstoff, und
- mindestens eine antioxidative Substanz.

2. Zusammensetzung nach Anspruch 1, **gekennzeichnet durch** eine Wasseraktivität a_{w} zwischen 0,2 und 0,5, bevorzugt zwischen 0,25 und 0,4.

3. Zusammensetzung nach einem der vorhergehenden Ansprüche, **dadurch gekennzeichnet, dass** Phospholipide aus dem Krillöl in einer Menge zwischen 20 und 35 Gew%, bevorzugt 23 und 33,5 Gew% enthalten sind.

4. Zusammensetzung nach einem der vorhergehenden Ansprüche, **dadurch gekennzeichnet, dass** das Silica in einer Menge zwischen 5 und 15 Gew%, bevorzugt zwischen 6 und 10 Gew% enthalten ist.

5. Zusammensetzung nach einem der vorhergehenden Ansprüche, **dadurch gekennzeichnet, dass** das Krillöl mittels Ultrazentrifugation bei 210.000 bis 240.000 g für 20 bis 30 Minuten vorbehandelt wird.

6. Zusammensetzung nach einem der vorhergehenden Ansprüche, **dadurch gekennzeichnet, dass** der mindestens eine wasserlösliche Wirkstoff Metallkationen, insbesondere Eisenkationen, oder organische Kationen umfasst.

7. Zusammensetzung nach einem der vorhergehenden Ansprüche, **dadurch gekennzeichnet, dass** der wasserlösliche Wirkstoff in einer Menge zwischen 0,05 und 20 Gew%, bevorzugt zwischen 0,1 und 10 Gew%, insbesondere bevorzugt zwischen 0,2 und 8,6 Gew% enthalten ist.

8. Zusammensetzung nach einem der vorhergehenden Ansprüche, **dadurch gekennzeichnet, dass** der mindestens eine fettlösliche Wirkstoff fettlösliche Nahrungsbestandteile oder Arzneistoffe umfasst.

9. Zusammensetzung nach einem der vorhergehenden Ansprüche, **dadurch gekennzeichnet, dass** der fettlösliche Wirkstoff ausgewählt ist aus der Gruppe enthaltend Coenzym Q10.

10. Zusammensetzung nach einem der vorhergehenden Ansprüche, **dadurch gekennzeichnet, dass** der fettlösliche Wirkstoff in einer Menge zwischen 5 und 25 Gew%, bevorzugt zwischen 10 und 23 Gew% enthalten ist.

11. Zusammensetzung nach einem der vorhergehenden Ansprüche **gekennzeichnet durch** mindestens eine fettlösliche antioxidative Substanz ausgewählt aus einer Gruppe enthaltend Substanzen mit Vitamin E-Aktivität, insbesondere Tocopherole und Trienole, und Carotinoiden.

12. Zusammensetzung nach einem der vorhergehenden Ansprüche umfassend aus Krillöl gewonnene Phospholipide, insbesondere Phosphatidylcholin, Silica, Eisen(II)sulfat, Tocopherole und Sojaöl.

13. Zusammensetzung nach einem der Ansprüche 1 bis 11 umfassend aus Krillöl gewonnene Phospholipide, insbesondere Phosphatidylcholin, Silica, Coenzym Q10, Tocopherole, Trienole, Sojaöl und Carotinoide.

14. Verfahren zur Herstellung einer Zusammensetzung gemäß einem der Ansprüche 1 bis 13 umfassend die Schritte:
- Vorbehandeln von Krillöl mittels Ultrazentrifugation bei 200.000 bis 250.000 g für 20 bis 30 Minuten;
- Vermischen des vorbehandelten Krillöls mit mindestens einem wasserlöslichen Wirkstoff und/oder mindestens einem fettlöslichen Wirkstoff;
- Zugabe von Silica und mindestens einer antioxidativen Substanz,
- Rühren der Mischung aus vorbehandelten Krillöl, wasserlöslichem Wirkstoff und/oder fettlöslichem Wirkstoff, Silica und antioxidativer Substanz über einen vorgegebenen Zeitraum, und
- Zentrifugation der Mischung bei 14.000 bis 16.000 g bei gleichzeitiger Absenkung der Temperatur der Mischung auf einen Wert zwischen 3°C und 10°C, bevorzugt zwischen 5°C und 8°C.

15. Verfahren nach Anspruch 14, **dadurch gekennzeichnet, dass** das vorbehandelte Krillöl vor dem Vermischen mit dem mindestens einen Wirkstoff, bevorzugt in einer Inertgas-Atmosphäre, auf eine Temperatur zwischen 40°C und 90°C bevorzugt zwischen 60°C und 80°C erwärmt wird.

## Claims

1. A substantially anhydrous composition comprising
- Krill oil pretreated by ultracentrifugation, the krill oil being pretreated by ultracentrifugation at 200,000 to 250,000 g for 20 to 30 minutes.
- optionally silica as emulsifier,
- at least one water-soluble active substance and/or at least one fat-soluble active substance, and
- at least one antioxidant substance.

2. Composition according to claim 1, **characterized by** a water activity a_{w} between 0.2 and 0.5, preferably between 0.25 and 0.4.

3. Composition according to one of the preceding claims, **characterized in that** phospholipids from the krill oil are present in an amount between 20 and 35 % by weight, preferably 23 and 33.5 % by weight.

4. Composition according to one of the preceding claims, **characterized in that** the silica is present in an amount between 5 and 15% by weight, preferably between 6 and 10% by weight.

5. Composition according to one of the preceding claims, **characterized in that** the krill oil is pretreated by ultracentrifugation at 210,000 to 240,000 g for 20 to 30 minutes.

6. Composition according to one of the preceding claims, **characterized in that** the at least one water-soluble active substance comprises metal cations, in particular iron cations, or organic cations.

7. Composition according to one of the preceding claims, **characterized in that** the water-soluble active substance is present in an amount between 0.05 and 20% by weight, preferably between 0.1 and 10% by weight, in particular preferably between 0.2 and 8.6% by weight.

8. Composition according to one of the preceding claims, **characterized in that** the at least one fat-soluble active substance comprises fat-soluble food components or drugs.

9. Composition according to one of the preceding claims, **characterized in that** the fat-soluble active substance is selected from the group comprising coenzyme Q10.

10. Composition according to one of the preceding claims, **characterized in that** the fat-soluble active substance is contained in an amount between 5 and 25% by weight, preferably between 10 and 23% by weight.

11. Composition according to one of the preceding claims **characterized by** at least one fat-soluble antioxidant substance selected from a group comprising substances having vitamin E activity, in particular tocopherols and trienols, and carotenoids.

12. Composition according to one of the preceding claims comprising phospholipids derived from krill oil, in particular phosphatidylcholine, silica, ferrous sulphate, tocopherols and soybean oil.

13. Composition according to any one of Claims 1 to 11 comprising phospholipids derived from krill oil, in particular phosphatidylcholine, silica, coenzyme Q10, tocopherols, trienols, soybean oil and carotenoids.

14. A process for preparing a composition according to any one of Claims 1 to 13 comprising the steps:
- Pretreating krill oil by ultracentrifugation at 200,000 to 250,000 g for 20 to 30 minutes;
- Mixing the pretreated krill oil with at least one water-soluble active substance and/or at least one fat-soluble active substance;
- Addition of silica and at least one antioxidant substance,
- stirring the mixture of pre-treated krill oil, water-soluble active substance and/or fat-soluble active substance, silica and antioxidant substance over a predetermined period of time, and
- Centrifugation of the mixture at 14,000 to 16,000 g with simultaneous reduction of the temperature of the mixture to a value between 3°C and 10°C, preferably between 5°C and 8°C.

15. Process according to claim 14, **characterized in that** the pretreated krill oil is heated to a temperature between 40°C and 90°C, preferably between 60°C and 80°C, prior to mixing with the at least one active ingredient, preferably in an inert gas atmosphere.

## Revendications

1. Composition sensiblement sans eau comprenant
- de l'huile de krill prétraitée par ultracentrifugation, dans laquelle l'huile de krill est prétraitée par ultracentrifugation de 200 000 à 250 000 g pendant 20 à 30 minutes,
- en option de la silice en tant qu'agent émulsifiant,
- au moins un principe actif hydrosoluble et/ou au moins un principe actif liposoluble, et
- au moins une substance antioxydante.

2. Composition selon la revendication 1, **caractérisée par** une activité de l'eau a_{w} entre 0,2 et 0,5, de manière préférée entre 0,25 et 0,4.

3. Composition selon l'une quelconque des revendications précédentes, **caractérisée en ce que** des phospholipides issus de l'huile de krill sont contenus en une quantité entre 20 et 35 % en poids, de manière préférée 23 et 33,5 % en poids.

4. Composition selon l'une quelconque des revendications précédentes, **caractérisée en ce que** la silice est contenue en une quantité entre 5 et 15 % en poids, de manière préférée entre 6 et 10 % en poids.

5. Composition selon l'une quelconque des revendications précédentes, **caractérisée en ce que** l'huile de krill est prétraitée par ultracentrifugation de 210 000 à 240 000 g pendant 20 à 30 minutes.

6. Composition selon l'une quelconque des revendications précédentes, **caractérisée en ce que** l'au moins un principe actif hydrosoluble comprend des cations métalliques, en particulier des cations de fer, ou des cations organiques.

7. Composition selon l'une quelconque des revendications précédentes, **caractérisée en ce que** le principe actif hydrosoluble est contenu en une quantité entre 0,05 et 20 % en poids, de manière préférée entre 0,1 et 10 % en poids, en particulier de manière préférée entre 0,2 et 8,6 % en poids.

8. Composition selon l'une quelconque des revendications précédentes, **caractérisée en ce que** l'au moins un principe actif liposoluble comprend des nutriments liposolubles ou des médicaments.

9. Composition selon l'une quelconque des revendications précédentes, **caractérisée en ce que** le principe actif liposoluble est choisi parmi le groupe contenant une coenzyme Q10.

10. Composition selon l'une quelconque des revendications précédentes, **caractérisée en ce que** le principe actif liposoluble est contenu en une quantité entre 5 et 25 % en poids, de manière préférée entre 10 et 23 % en poids.

11. Composition selon l'une quelconque des revendications précédentes, **caractérisée par** au moins une substance antioxydante liposoluble choisie parmi un groupe contenant des substances avec une activité de vitamine E, en particulier des tocophérols et des triénols, et des caroténoïdes.

12. Composition selon l'une quelconque des revendications précédentes comprenant des phospholipides obtenus à partir d'huile de krill, en particulier de la phosphatidylcholine, de la silice, du sulfate de fer (II), des tocophérols et de l'huile de soja.

13. Composition selon l'une quelconque des revendications 1 à 11 comprenant des phospholipides obtenus à partir d'huile de krill, en particulier de la phosphatidylcholine, de la silice, une coenzyme Q10, des tocophérols, des triénols, de l'huile de soja et des caroténoïdes.

14. Procédé servant à produire une composition selon l'une quelconque des revendications 1 à 13, comprenant les étapes de :
- prétraitement de l'huile de krill par ultracentrifugation de 200 000 à 250 000 g pendant 20 à 30 minutes ;
- mélange de l'huile de krill prétraitée à au moins un principe actif hydrosoluble et/ou au moins un principe actif liposoluble ;
- ajout de silice et d'au moins une substance antioxydante ;
- agitation du mélange composé d'huile de krill prétraitée, d'un principe actif hydrosoluble et/ou d'un principe actif liposoluble, de silice et d'une substance antioxydante sur une durée prédéfinie ; et
- centrifugation du mélange de 14 000 à 16 000 g tout en abaissant simultanément la température du mélange à une valeur entre 3 °C et 10 °C, de manière préférée entre 5 °C et 8 °C.

15. Procédé selon la revendication 14, **caractérisé en ce que** l'huile de krill prétraitée est chauffée avant le mélange à l'au moins un principe actif, de manière préférée sous une atmosphère de gaz inerte, à une température entre 40 °C et 90 °C, de manière préférée entre 60 °C et 80 °C.
